# EUROPÄISCHE PATENTSCHRIFT

(11) **EP 1 604 607 B1**
(45) Veröffentlichungstag und Bekanntmachung des Hinweises auf die Patenterteilung: **04.10.2006**
(21) Anmeldenummer: 05011624.3
(22) Anmeldetag: 30.05.2005
(51) Int. Cl.: A61B 1/005

(54) **Biegbarer Abschnitt eines Einführtubus eines Endoskopes und Verfahren zu dessen Herstellung**
Flexible section of an insertion tube of an endoscope and method of manufacturing thereof
Partie flexible d'un tube d'introduction d'un endoscope et procédé de fabrication

(30) Priorität: 08.06.2004 DE 102004027850
(43) Veröffentlichungstag der Anmeldung: 14.12.2005
(73) Patentinhaber: Henke-Sass, Wolf GmbH, D-78532 Tuttlingen (DE)
(72) Erfinder: Brunnen, Rainer Dirk, 78606 Seitingen-Oberflacht (DE); Simon, Thomas Jürgen, 78576 Emmingen (DE)
(74) Vertreter: Geyer, Fehners & Partner

(56) Entgegenhaltungen:
- EP-A- 0 626 604
- EP-A- 0 782 836
- DE-A1- 19 535 179
- DE-C1- 10 113 713

## Beschreibung

Die Erfindung bezieht sich auf- einen biegbaren Abschnitt, der am distalen Ende eines Einführtubus eines Endoskopes angeordnet ist, umfassend eine Mehrzahl von Rohrsegmenten, wobei jedes dieser Rohrsegmente Verbindungsmittel aufweist, die mit den Verbindungsmitteln des benachbarten Rohrsegmentes zusammenwirken, sowie Steuerzüge, mit denen die Abbiegung des biegbaren Abschnittes steuerbar ist, und auf ein Verfahren zur Herstellung eines solchen biegbaren Abschnittes.

Endoskope mit solchen biegbaren und steuerbaren Abschnitten an den distalen Enden ihres Einführtubus werden in einer großen Anzahl von Druckschriften beschrieben, einige davon sollen nachstehend näher erörtert werden, z. B. die DE 101 43 966 A1, DE 42 34 833 A1, EP 0 439 931 B1, EP 1 090 581 B1, EP 0 626 604 A2 und EP 0 782 836 A1.

Alle diese genannten Druckschriften zeigen biegbare Abschnitte, die jeweils an den distalen Enden eines Einführtubus eines Endoskopes vorgesehen sind, wobei der Einführtubus starr oder flexibel ausgebildet sein kann. Eine wesentliche Eigenschaft des biegbaren Abschnittes des Einführtubus aber ist, daß dieser hinsichtlich seiner Abbiegungsrichtung steuerbar ist, und zwar über in dem Einführtubus integrierte Steuerzüge.

In der EP 1 090 581 B1 ist ein biegbarer Abschnitt beschrieben, der über zwei Steuerzüge verfügt, d. h., der biegbare Abschnitt ist nur in einer Ebene abbiegbar, wohingegen die übrigen vorgenannten Druckschriften biegbare Abschnitte mit jeweils vier Steuerzügen zeigen, die das Abbiegen des biegbaren Abschnittes in zwei Ebenen und infolge dessen ein räumliches Abbiegen erlauben.

Alle diese bekannten biegbaren Abschnitte bestehen aus einer Mehrzahl von Verbindungs- bzw. Gelenkringen, allgemeiner gesagt, aus Rohrsegmenten, die miteinander über Verbindungsmittel gegeneinander verschwenkbar miteinander verbunden sind, wobei diese Verbindungsmittel jeweils an den Stirnseiten der Rohrsegmente angeordnet sind.

Die Anordnung der Verbindungsmittel an den jeweiligen Stirnseiten der einzelnen Rohrsegmente ist nun davon abhängig, ob der biegbare Abschnitt nur in einer Ebene abbiegbar sein soll oder ob er räumlich, also in zwei Ebenen abbiegbar sein soll. Soll der biegbare Abschnitt nur, wie in der EP 1 090 581 B1 dargestellt, in einer Ebene abbiegbar sein, dann sind an jeder Stirnseite, also in distaler oder in proximaler Richtung gesehen, jeweils zwei Verbindungsmittel angeordnet, wobei sich diese Verbindungsteile um 180° in Umfangsrichtung versetzt gegenüberliegen. Die Schwenkachsen dieser Verbindungsmittel an den einzelnen Rohrsegmenten verlaufen jeweils, in axialer Richtung gesehen, auf einer durchgehenden Mantellinie.

Für den Fall, daß der biegbare Abschnitt allerdings räumlich abbiegbar sein soll, müssen die einzelnen Rohrsegmente auch in einer zweiten Ebene, die um 90° versetzt zu der ersten Ebene angeordnet ist, gegeneinander verschwenkbar sein, dazu ist es erforderlich, daß die jeweils an den Stirnseiten eines Rohrsegmentes angeordneten Verbindungsmittel gegeneinander um 90° versetzt angeordnet sind, wie beispielsweise der EP 0 439 931 B1, EP 0 626 604 A2 und EP 0 782 836 A1 zu entnehmen ist. Damit diese räumliche Abbiegung auch gesteuert werden kann, sind selbstverständlich vier Steuerzüge angeordnet, die jeweils 90° zueinander umfangsmäßig versetzt angeordnet sind.

Die Verbindungsmittel sind als über die jeweiligen Stirnseiten der Rohrsegmente vorstehende Laschen ausgebildet, die beispielsweise Bohrungen aufweisen, in welche radial einstehende Bolzen eingeführt sind. Dies bedeutet, daß diese Verbindungsmittel, speziell die laschenförmigen Vorsprünge, sich überlappen müssen und deswegen nach außen oder nach innen aus der Mantelfläche der Rohrsegmente abgebogen sind. Diese Verbindungsmittel wie auch die darin angeordneten Bolzen stehen dann aber über den Außen- und/oder Innenumfang des biegbaren Abschnittes hervor, insbesondere aber ragen die Bolzen in den Innenraum des biegbaren Abschnittes hinein.

Die Steuerzüge werden dabei im Inneren des biegbaren Abschnittes geführt, und zwar, wie in der DE 42 34 833 A1, EP 1 090 581 B1, EP 0 626 604 A2 und EP 0 782 836 A1 gezeigt, in Führungen, die an den Innenseiten einzelner Rohrsegmente vorgesehen sind. Bei der DE 101 43 966 A1 wie auch der EP 0 439 931 B1 sind solche Führungen unmittelbar an den in den Innenraum des biegbaren Abschnittes einstehenden Bolzenköpfen der Verbindungsmittel ausgebildet, ähnlich wie Ringösen.

Allen diesen bekannten biegbaren Abschnitten ist gemein, daß sie aus Rohrsegmenten und Verbindungsteilen bestehen, die äußerst aufwendig hinsichtlich ihrer Herstellung, insbesondere aber auch bezüglich ihrer Montage sind und es ist deshalb ein erstes Ziel der hier vorliegenden Erfindung, diesen Herstellungs- und Montageprozeß wesentlich zu vereinfachen, sowie den Innenraum des biegbaren Abschnittes glattwandig und frei von Vorsprüngen auszubilden.

Es ist aber auch ein weiteres Ziel der Erfindung, den Innenraum frei von Steuerzügen auszubilden, die sonst den Einbau von ebenfalls erforderlichen Leuchtmitteln, beispielsweise Glasfasersträngen und elektrischen Leitungen für die Optik- und Sensoreinheit am distalen Ende des biegbaren Abschnittes behindern. Insbesondere soll der Innenraum aber auch für Installationen freigehalten werden, die für Spül- und Absaugvorgänge und gegebenenfalls am distalen Ende wirkende operative Werkzeuge benötigt werden.

Offenbart wird zudem ein Verfahren zur Herstellung eines solchen biegbaren Abschnittes aus einem vorgegebenen biegesteifen Rohr das es ermöglicht, daß die einzelnen Rohrsegmente mit ihren Verbindungsmitteln gar nicht mehr unter Verwendung weiterer Verbindungsmittel, beispielsweise Bolzen und Nieten etc., zusammengefügt werden müssen, sondern, sozusagen in einem Verfahrensschritt, gebrauchsfertig und weiterverwendbar sind.

Das erste Ziel wird bei einem biegbaren Abschnitt der eingangs beschriebenen Art dadurch erreicht, daß die jeweiligen Verbindungsmittel eines Rohrsegmentes an dessen Stirnseiten in axialer Richtung vorstehend und innerhalb des Mantels des Rohrsegmentes liegend ausgebildet sind und die Dicke des Mantels nicht überschreiten, wobei die Verbindungsmittel, die an den jeweiligen Stirnseiten benachbarter Rohrsegmente vorgesehen sind und sich gegenüberliegen, sich nach Art einer scharnierförmigen Verbindung ergänzen.
Diese erfindungsgemäßen Verbindungsmittel stehen weder nach außen noch nach innen über die äußere bzw. innere Mantelfläche der Rohrsegmente hervor und es sind auch keine zusätzlichen Mittel vorgesehen, die die Verbindungsmittel aneinanderhalten und dadurch Vorsprünge bilden. Es kann also auf bolzen- und/oder nietenförmige Mittel, die radial ausgerichtete Schwenkachsen bilden und die jeweiligen Verbindungsmittel miteinander schwenkbar verbinden, verzichtet werden. Die Verbindungsmittel sind vielmehr innerhalb der Manteldicke der Rohrsegmente ausgebildet und stehen in axialer Richtung ineinander ein und sind gegeneinander verschwenkbar. Durch diese erfindungsgemäße Ausbildung wird dafür Sorge getragen, daß sowohl die äußere als auch die innere Mantelfläche der Rohrsegmente und damit insbesondere der Innenraum des biegbaren Abschnittes keinerlei Vorsprünge aufweist, es entstehen glattflächige Außen- und Innenseiten.

In erfindungsgemäßer Weiterbildung sind die an der einen Stirnseite eines Rohrsegmentes ausgebildeten Verbindungsmittel nach Art eines zur Stirnseite eines benachbarten Rohrsegmentes gerichtete und vorstehende Zapfen ausgebildet, und sind die an der anderen Stirnseite des Rohrsegmentes angeordneten Verbindungsmittel als den Zapfen eines benachbarten Rohrsegmentes umgreifende Klauen ausgebildet, wobei sowohl der Zapfen als auch die Klauen Teile des Mantels des jeweiligen Rohrsegmentes sind.
Diese vorbeschriebene Ausbildung der Verbindungsteile erlaubt es, eine die Rohrsegmente aneinander, haltende und gleichzeitig verschwenkbare Verbindung herzustellen, ohne daß zusätzliche Mittel in Form von Bolzen o. ä. eingesetzt werden müssen. Die klauenförmigen Verbindungsmittel bilden sozusagen eine Klammer zu dem zapfenförmigen Verbindungsmittel des anderen Rohrsegmentes, so daß ein die Rohrsegmente aneinander haltende Verbindung zustande kommt, ohne daß ein zusätzliches, separates Teil als Verbindungsmittel eingesetzt werden muß.

Vorteilhaft sind die jeweils an einer der Stirnseiten eines Rohrsegmentes angeordneten Verbindungsmittel entweder als Zapfen oder als Klauen ausgebildet, wobei dann die an der anderen Stirnseite desselben Rohrsegmentes vorgesehenen Verbindungsmittel umgekehrt entweder als Klauen oder als Zapfen ausgebildet sind.
Nach dieser Ausbildung sind die jeweils an einer bestimmten Stirnseite eines Rohrsegmentes vorgesehen Verbindungsmittel, die sich ja um 180° in Umfangsrichtung versetzt gegenüberliegen, identisch ausgebildet, entsprechend dann auch die dazu korrespondierenden zapfen- oder klauenförmigen Verbindungsmittel an der einer solchen Stirnseite eines Rohrsegmentes gegenüberliegenden Stirnseite eines anderen Rohrsegmentes. Diese jeweils identische Ausbildung der Verbindungsmittel an ein und derselben Stirnseite eines Rohrsegmentes vereinfacht die Herstellung solcher Rohrsegmente.

In erfindungsgemäßer Weiterbildung ist der an der Stirnseite eines Rohrsegmentes angeordnete Zapfen nach Art einer kreisförmigen Scheibe ausgebildet und schließen die an der Stirnseite des benachbarten Rohrsegmentes angeordneten Klauen eine kreisförmige Ausnehmung zwischen sich ein, welche den Abmessungen des kreisscheibenförmigen Zapfens entspricht, und wobei die Klauen den Zapfen in dieser Ausnehmung aufnehmen.
Eine solche Formgebung der Verbindungsmittel gewährleistet, daß diese innerhalb der Abmessungen des Mantels des Rohrsegmentes liegen und gewährleistet insbesondere, daß die Klauen weder in axialer Richtung noch in radialer Richtung von dem Zapfen abgezogen werden können.

In weiterer vorteilhafter Ausbildung weisen das am proximalen Ende des biegbaren Abschnittes angeordnete erste Rohrsegment und das am distalen Ende des biegbaren Abschnittes angeordnete letzte Rohrsegment jeweils an ihren die Enden des biegbaren Rohrabschnittes bildenden Stirnseiten keine Verbindungsmittel nach Art von Zapfen und/oder Klauen auf.
Diese jeweiligen ersten und letzten Rohrsegmente des biegbaren Abschnittes sind jeweils ihren entsprechenden Bestimmungen gemäß ausgebildet. So ist das letzte Rohrsegment am distalen Ende des biegbaren Abschnittes zur Aufnahme einer Optik und eines Sensors etc. und insbesondere zum Eindringen in eine Körperhöhle vorgesehen und unterliegt deshalb einer speziellen Ausbildung. Ebenso verhält es sich mit dem am proximalen Ende des biegbaren Abschnittes vorgesehenen Rohrsegment, dieses ist in einer besonderen und hier nicht beschriebenen Form mit dem proximalen Teil des rohrförmigen Einführtubus des Endoskopes verbunden.

Zur Erreichung des zweiten Zieles der Erfindung ist in dem biegbaren Abschnitt, im folgenden stets äußerer biegbarer Abschnitt genannt, ein hinsichtlich der Anzahl der Rohrsegmente und der Anordnung der Verbindungsteile an diesen Rohrsegmenten identisch ausgebildeter zweiter, innerer biegbarer Abschnitt eingesetzt, dessen Außendurchmesser dem Innendurchmesser des äußeren biegbaren Abschnittes so angepaßt ist, daß der innere biegbare Abschnitt in den äußeren biegbaren Abschnitt einschiebbar ist, wobei der innere biegbare Abschnitt in seiner äußeren Umfangsfläche mindestens zwei um 180° in Umfangsrichtung gegeneinander versetzt angeordnete und über die einzelnen Rohrsegmente in axialer Richtung verlaufende Nuten aufweist, in welchen jeweils ein Steuerzug geführt ist, wobei die beiden Nuten jeweils in Umfangsrichtung um 90° versetzt gegenüber den Verbindungsmitteln angeordnet sind.
Durch die besondere Ausbildung des biegbaren Abschnittes in einer sozusagen doppelwandigen Form, ergibt sich unter Ausbildung der Nuten in der äußeren Umfangsfläche des inneren biegbaren Abschnittes eine sichere Führung für die Steuerzüge, gleichzeitig wird erreicht, daß der Innenraum des doppelwandigen biegbaren Abschnittes, insbesondere also der Innenraum des inneren biegbaren Abschnittes frei bleibt von Seilzügen, wodurch das Einführen von sonstigen Schläuchen, elektrischen Leitungen, etc. sehr vereinfacht wird. Auch ist der Einbau der Seilzüge im doppelwandigen biegbaren Abschnitt verhältnismäßig einfach, müssen die Seilzüge doch nicht durch ösenförmige Führungen hindurchgeführt bzw. in diese eingefädelt werden, vielmehr können die Seilzüge beim Einschieben des inneren biegbaren Abschnittes in den äußeren biegbaren Abschnitt bereits in die jeweiligen Nuten eingelegt und in einem Arbeitsgang eingebaut, andererseits aber auch erst nach dem Zusammenbau des äußeren und inneren biegbaren Abschnittes in die Nuten eingeschoben werden.

Dadurch, daß die äußere Umfangsfläche des inneren biegbaren Abschnittes dicht an der inneren Umfangsfläche des äußeren biegbaren Abschnittes anliegt, bilden die Nuten mit der inneren Umfangsfläche des äußeren biegbaren Abschnittes eine geschlossene kanalförmige Führung, in der die Seilzüge ohne Klemmungen gleiten können.

In einer speziellen Ausbildung sind an der äußeren Umfangsfläche des inneren biegbaren Abschnittes vier axial verlaufende Nuten vorgesehen, die jeweils um 90° in Umfangsrichtung zueinander versetzt angeordnet sind, wobei gleichzeitig die an den jeweiligen Stirnseiten der Rohrsegmente paarweise angeordneten Verbindungsteile jeweils um 90° zueinander versetzt sind.
Die Anordnung von vier Seilzügen ist erforderlich, wenn der biegbare Abschnitt in zwei Ebenen, also räumlich biegbar sein soll, gleichzeitig müssen dann aber auch die Verbindungsmittel jeweils alternierend, in Umfangsrichtung um 90° versetzt zueinander an den jeweiligen Rohrsegmenten angeordnet sein, damit ein räumliches Abbiegen des biegbaren Abschnittes ermöglicht werden kann.

Damit die ineinandergesetzten inneren und äußeren biegbaren Abschnitte sich nicht etwa gegeneinander verschieben, wodurch die Biegbarkeit bzw. Verschwenkbarkeit der jeweiligen Rohrsegmente gegeneinander ver-, zumindest aber behindert werden könnte, sind die einzelnen Rohrsegmente der inneren und der äußeren Abschnitte, wenn sie sich exakt gegenüberliegen, also eine vollständige Kongruenz der biegbaren Abschnitte hergestellt ist, jeweils durch eine Punktschweißung aneinander fixiert.
Eine solche Punktschweißung ist einfach durchzuführen und garantiert die Unverschiebbarkeit der jeweiligen biegbaren Abschnitte zueinander.

Zudem wird ein Verfahren zur Herstellung eines biegbaren Abschnittes, der eine Vielzahl von Rohrsegmenten aufweist, welche in einer Reihe angeordnet sind und miteinander über Verbindungsteile gegeneinander schwenkbar miteinander verbunden sind, bereitgestellt, wobei das Verfahren folgende Schritte umfaßt:
a) Bereitstellen eines biegesteifen Rohres, das Abmessungen aufweist, die den gewünschten Abmessungen des biegbaren Abschnittes entspricht,
b) Bereitstellen einer Laser-Schneidevorrichtung,
c) Einsetzen des biegesteifen Rohres in die Laser-Schneidevorrichtung,
d) Führung des Laserstrahles der Laser-Schneidevorrichtung entsprechend den aufgrund der Konfiguration der Rohrsegmente und der jeweiligen Verbindungsmittel vorgegebenen Schnitt-Linien, wobei der Laserstrahl stets radial auf das Rohr gerichtet ist, und
e) Herausbrechen der aus dem biegesteifen Rohr herausgeschnittenen Wandteile zur Ermöglichung der Verschwenkbarkeit der einzelnen Rohrsegmente zueinander. Aufgrund der speziellen und vorstehend beschriebenen Ausbildung der Verbindungsmittel der einzelnen Rohrsegmente ist es möglich, das Verfahren durchzuführen und, in einem einzigen Schneideprozeß, den biegbaren Abschnitt aus einem biegesteifen Rohr herauszuschneiden. Hierbei wird der Fachmann einen Laserstrahl mit der geeigneten Dicke wählen, um das einwandfreie Funktionieren der Verbindungsmittel, insbesondere deren Verschwenkbarkeit zu gewährleisten. Nach dem Schneideprozeß ist der biegbare Abschnitt bereits einsetzbar bzw. kann in das herzustellende Endoskop eingebaut werden. Durch dieses Verfahren wird insbesondere der eingangs beschriebene umständliche Montageprozeß vermieden, bei dem einzelne Rohrsegmente miteinander über zusätzliche Verbindungsmittel wie Bolzen, Zapfen, Nieten o. ä. miteinander verbunden werden müssen.

In einer vorteilhaften Ausbildung des vorbeschriebenen Verfahrens wird das biegesteife Rohr in der Schneidevorrichtung unter dem ortsfesten Laserstrahl gedreht und/oder axial verschoben.
Diese Form der Durchführung des Verfahrens erlaubt eine einfachere Konstruktion der Laserschneidevorrichtung.

Schließlich können in weiterer vorteilhafter Weise die aus dem Rohr herausgeschnittenen Wandteile mittels Ultraschall aus diesem herausgebrochen werden.
Dieses Verfahren weist den Vorteil auf, daß keine mechanisch wirkenden Werkzeuge eingesetzt werden müssen.

Eine die Erfindung nicht beschränkende und diese nur beispielhaft beschreibende Ausführungsform wird anhand der nachstehend genannten Figuren erläutert.

### Es zeigen:

- Fig. 1:: eine Seitenansicht auf ein Endoskop, speziell ein Nasopharyngoskop
- Fig. 1A:: eine etwas vergrößerte Ansicht des distalen Endes eines Einführtubus,
- Fig. 1B:: eine Ansicht auf das distale Ende gemäß Fig. 1A in einer um ca. 125° abgebogenen Stellung,
- Fig. 2A:: eine nochmals vergrößerte Ansicht auf das distale Ende gemäß Fig. 1A, versehen mit einer teilweise aufgebrochenen schlauchförmigen Kunststoffumhüllung,
- Fig. 2B:: eine Ansicht auf das distale Ende gemäß Fig. 2A, jedoch in einer abgebogenen Stellung,
- Fig. 3:: eine Ansicht auf ein biegesteifes Rohr vor der Ausbildung zu einem äußeren biegbaren Abschnitt des Einführtubus,
- Fig. 4:: eine Ansicht auf ein biegesteifes Rohr vor der Ausbildung zu einem inneren biegbaren Abschnitt des Einführtubus,

- Fig. 5A:: eine Draufsicht auf den äußeren biegbaren Abschnitt,
- Fig. 5B:: einen Längsschnitt gemäß Linie B-B in Fig. 5A,
- Fig. 5C:: eine Stirnansicht auf den äußeren biegbaren Abschnitt,
- Fig. 5D:: eine perspektivische Seitenansicht des äußeren biegbaren Abschnitts gemäß Fig. 5A,
- Fig. 6A:: eine Draufsicht auf den inneren biegbaren Abschnitt des Einführtubus,
- Fig. 6B:: einen Schnitt durch den inneren biegbaren Abschnitt gemäß Linie B-B in Fig. 6A,
- Fig. 6C:: eine Stirnansicht auf den inneren biegbaren Abschnitt,
- Fig. 6D:: eine perspektivische Seitenansicht des inneren biegbaren Abschnitts gemäß Fig. 6A,
- Fig. 7A:: eine Draufsicht auf das biegbare distale Ende des Einführtubus, bestehend aus äußerem und innerem biegbaren Abschnitt,
- Fig. 7B:: einen Schnitt gemäß Linie B-B durch das biegbare distale Ende gemäß Fig. 7A,
- Fig. 7C:: eine Stirnansicht auf das biegbare distale Ende mit äußerem und innerem biegbaren Abschnitt,
- Fig. 7D:: eine perspektivische Seitenansicht auf das biegbare distale Ende gemäß Fig. 7A,
- Fig. 8:: eine perspektivische Darstellung auf ein einzelnes Rohrsegment des äußeren biegbaren Abschnitts,
- Fig. 8A:: eine Draufsicht auf das Rohrsegment gemäß Fig. 8,
- Fig. 8B:: einen Schnitt gemäß Linie B-B in Fig. 8A durch das Rohrsegment,
- Fig. 8C:: eine Stirnansicht auf das Rohrsegment,
- Fig. 8D:: eine vergrößerte Darstellung des Details D gemäß Fig. 8B,
- Fig. 9:: eine perspektivische Seitenansicht auf ein Rohrsegment des äußeren Abschnitts, bei dem die Verbindungsmittel an einer ersten Stirnseite um 90° zu den Verbindungsmitteln an der anderen Stirnseite versetzt angeordnet sind,
- Fig. 9A:: eine Draufsicht auf das Rohrsegment gemäß Fig. 9,
- Fig. 9B:: einen Schnitt gemäß Linie B-B in Fig. 9A durch das Rohrsegment,
- Fig. 9C:: eine Stirnansicht des Rohrsegmentes,
- Fig. 9D:: eine vergrößerte Darstellung des Details D in Fig. 9B
- Fig. 10:: eine perspektivische Seitenansicht auf ein Rohrsegment des inneren biegbaren Abschnitts mit entsprechender Anordnung der zueinander versetzten Verbindungsmittel gemäß Fig. 9,
- Fig. 10A:: eine Draufsicht auf das Rohrsegment gemäß Fig. 10,
- Fig. 10B:: einen Schnitt durch das Rohrsegment gemäß Linie B-B in Fig. 10A,
- Fig. 10C:: eine Stirnansicht auf das Rohrsegment,
- Fig. 10D:: eine Ansicht der gegenüberliegenden Stirnseite des Rohrsegmentes,
- Fig. 11A:: eine Draufsicht auf einen inneren biegbaren Abschnitt für 4-fach Seilzüge,
- Fig. 11B:: einen Schnitt durch den inneren biegbaren Abschnitt gemäß Linie B-B in Fig. 11A,
- Fig. 11C:: eine Stirnansicht auf den inneren biegbaren Abschnitt,

- Fig. 11D:: eine perspektivische Seitenansicht auf den inneren biegbaren Abschnitt für 4-fach Seilzüge,
- Fig. 12:: Darstellung der Laserschnittlinie in abgewickelter Form,
- Fig. 13:: Darstellung der aus dem Rohr herauszubrechenden freigeschnittenen Wandteile und
- Fig. 14:: Darstellung eines biegbaren Abschnitts mit Abstands -und Winkelangaben.

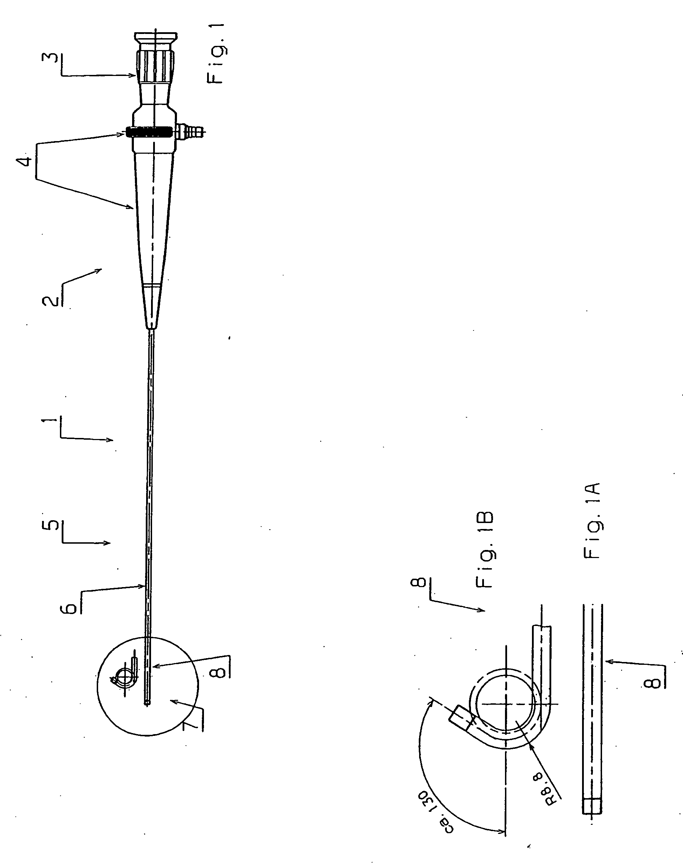

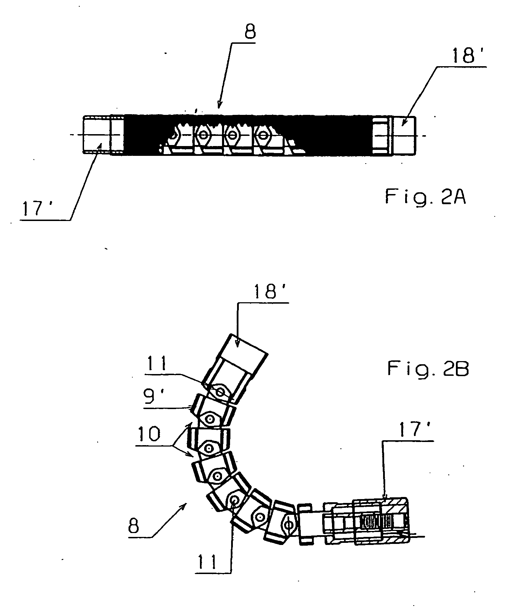

Das in Fig. 1 dargestellte Endoskop 1 besteht im wesentlichen aus einem zur Handhabung und Betätigung bestimmten proximalen Abschnitt 2, der ein Okular 3 und ein Handbetätigungsteil 4 aufweist, und weiterhin aus einem zum Einführen in eine Körperhöhle vorgesehenen distalen Abschnitt 5, der als Einführtubus 6 ausgebildet ist und welcher an seinem distalen Ende 7 einen biegbaren Abschnitt 8 aufweist. Dieser biegbare Abschnitt 8 unterscheidet sich von einem beispielsweise flexibel ausgebildeten Einführtubus 6 dadurch, daß er steuerbar abbiegbar ist, also vom Handbedienungsteil 4 aus in gewünschte Richtungen abgebogen werden kann, wie in Figuren 1A und 1B dargestellt. Kann der Einführtubus 6 in einfacher Weise als Kunststoffschlauch ausgebildet sein, so ist dies bei dem steuerbaren biegbaren Abschnitt nicht möglich, der biegbare Abschnitt muß, wie den Figuren 2A und 2B zu entnehmen ist, aus einzelnen, sich gegeneinander verstellbaren Rohrsegmenten 9 hergestellt sein, die über Steuerzüge 10 in die gewünschte Richtung verstellbar sind, wobei die Rohrsegmente 9 über Verbindungsmittel 11 miteinander verschwenkbar verbunden sind.

Gegenüber den eingangs beschriebenen bekannten biegbaren Abschnitten von Endoskopen sind die in diesem Ausführungsbeispiel beschriebenen Rohrsegmente 9 doppelwandig ausgebildet, d. h., sie bestehen aus einem äußeren und einem inneren, jeweils zueinander identisch ausgebildeten Rohrsegment 9' bzw. 9", die lediglich unterschiedliche Durchmesser und gegebenenfalls auch unterschiedliche Wanddicken aufweisen und in einfacher Weise ineinander geschoben werden.

Da diese äußeren und inneren Rohrsegmente 9' bzw. 9" im wesentlichen hinsichtlich ihrer Konfiguration identisch ausgebildet sind, wird im weiteren zunächst die Ausbildung eines solchen Rohrsegmentes am Beispiel eines äußeren Rohrsegmentes 9', also am Beispiel eines aus solchen Rohrsegmenten 9' gebildeten äußeren biegbaren Abschnittes 8' des Einführtubus des Endoskopes 1 beschrieben.

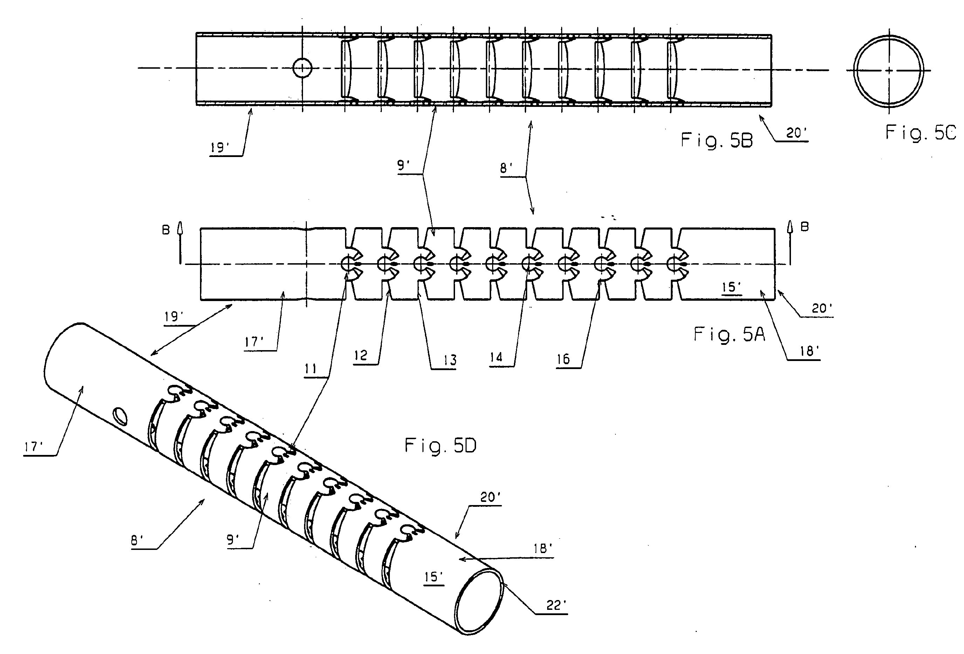

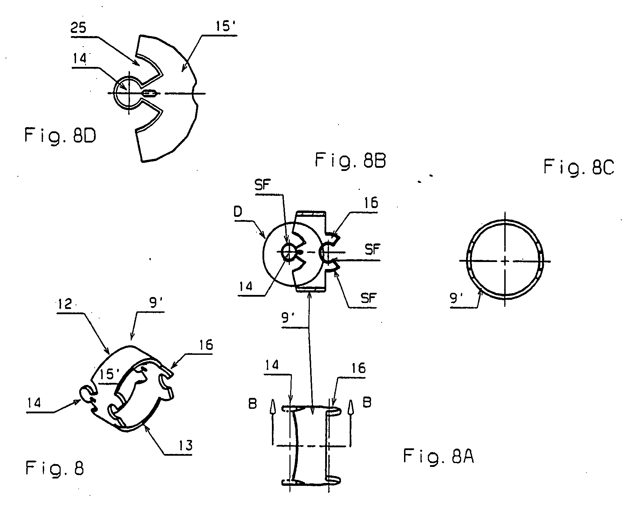

Wie den Figuren 5A bis 5D und den Figuren 8 bis 8D zu entnehmen ist, weisen die einzelnen Rohrsegmente 9' an ihren jeweiligen Stirnseiten 12 bzw. 13 in axialer Richtung vorstehende Verbindungsmittel 11 auf, wobei diese Verbindungsmittel 11 zwei unterschiedliche Ausführungsformen aufweisen. So ist das an der Stirnseite 12 eines Rohrsegmentes 9' vorstehende Verbindungsmittel 11 als Zapfen 14 ausgebildet, der nach Art einer kreisförmigen Scheibe aus dem Mantel 15' des Rohrsegmentes 9' herausgeschnitten ist.

Das an der gegenüberliegenden Stirnseite 13 vorgesehene Verbindungsmittel 11 ist dagegen als den Zapfen 14 umgreifende Klauen 16 ausgebildet, die paarweise an der Stirnseite 13 vorgesehen sind. Diese Klauen 16 bilden zwischen sich eine kreisförmige Ausnehmung aus, die die Abmessungen des kreisscheibenförmigen Zapfens 14 aufweisen. Auch diese Klauen 16 sind aus dem Mantel 15' des Rohrsegmentes 9' herausgeschnitten. Dies bedeutet, daß die Verbindungsmittel 11, seien sie als Klauen 16 oder als Zapfen 14 ausgebildet, innerhalb des Außen- bzw. Innenumfanges des biegbaren Abschnittes 8' liegen, also weder nach innen noch nach außen hervorstehen und damit die Dicke des Mantels 15' eines Rohrsegmentes 9' bzw. des biegbaren Abschnittes 8' nicht überschreiten. Dies hat zur Folge, daß sowohl die Innenwandung als auch die Außenwandung des biegbaren Abschnittes glattflächig ausgebildet ist.

Lediglich die proximalen bzw. distalen Endabschnitte 17' bzw.18' des biegbaren Abschnittes 8' weisen nur an den jeweiligen, den Rohrsegmenten zugewandten Stirnseiten entsprechend ausgebildete Verbindungsmittel auf. Die die eigentlichen Enden 19' bzw. 20' bildenden Stirnseiten 21' bzw. 22' weisen keine Verbindungsmittel nach Art von Zapfen und/oder Klauen auf.

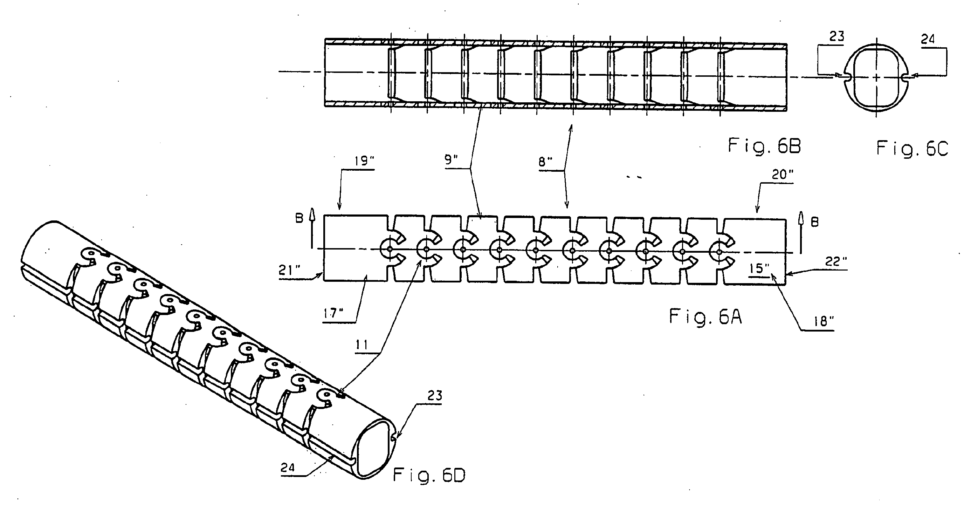

In den Figuren 6A bis 6B ist der in den äußeren biegbaren Abschnitt 8' gemäß Figuren 5A bis 5D einzuschiebende innere biegbare Abschnitt 8" entsprechend dargestellt, dieser unterscheidet sich hinsichtlich der Ausbildung seiner Rohrsegmente 9" und Verbindungsmittel 11 nicht gegenüber dem äußeren biegbaren Abschnitt 8', er weist lediglich einen geringeren Außendurchmesser auf, der nahezu dem Innendurchmesser des äußeren biegbaren Abschnittes 8' entspricht, wodurch gewährleistet wird, daß der innere biegbare Abschnitt 8" in den äußeren biegbaren Abschnitt 8' hineingeschoben werden kann.

Allerdings weist der innere biegbare Abschnitt 8" in seiner äußeren Umfangsfläche mindestens zwei um 180° in Umfangsrichtung gegeneinander versetzt angeordnete und über die einzelnen Rohrsegmente 9" in axialer Richtung verlaufende Nuten 23 bzw. 24 auf, in welchen die hier nicht dargestellten Steuerzüge 10 verlaufen.

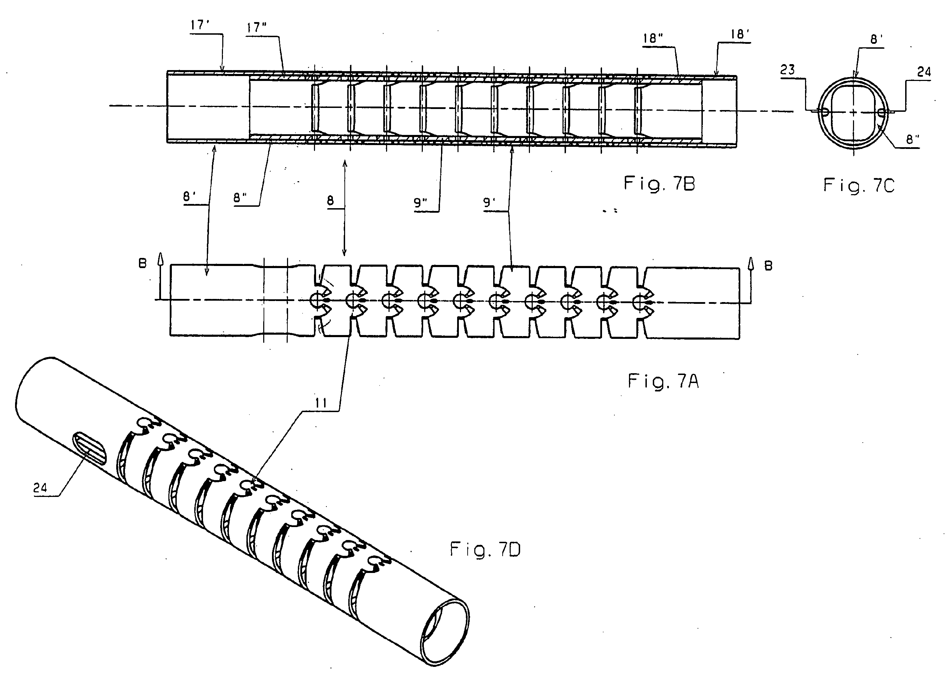

Die Figuren 7A bis 7D zeigen den biegbaren Abschnitt 8, bestehend aus dem äußeren biegbaren Abschnitt 8' und dem darin eingesetzten inneren biegbaren Abschnitt 8". Insbesondere der Fig. 7B ist zu entnehmen, daß die Gesamtlänge des biegbaren inneren Abschnittes 8" etwas kürzer ist als die Gesamtlänge des äußeren biegbaren Abschnittes 8', dies liegt daran, daß die proximalen bzw. distalen Endabschnitte 17" bzw. 18" kürzer ausgebildet sind als die entsprechenden Endabschnitte 17' bzw. 18' des äußeren biegbaren Abschnitts 8'. Dieser Unterschied hängt lediglich von rein konstruktiven Bedingungen ab, die mit den unterschiedlichen Funktionen der Endabschnitte im Zusammenhang stehen und hier keine Rolle spielen.

In den Figuren 8A bis 8D ist ein einzelnes Rohrsegment 9' des äußeren biegbaren Abschnittes 8' gezeigt, dem die besondere Ausbildung der zapfen- bzw. klauenförmigen Verbindungsmittel 14 bzw. 16 zu entnehmen ist. Um die Rohrsegmente 9' möglichst eng hintereinander anordnen zu können, also ohne besonders große Abstände zwischen ihnen vorsehen zu müssen, sind im Mantel 15' des Rohrsegmentes 9' Ausnehmungen 25 vorgesehen, in welche die als Klauen 16 ausgebildeten Verbindungsmittel 11 des gegenüberliegenden Rohrsegmentes 9' einstehen.

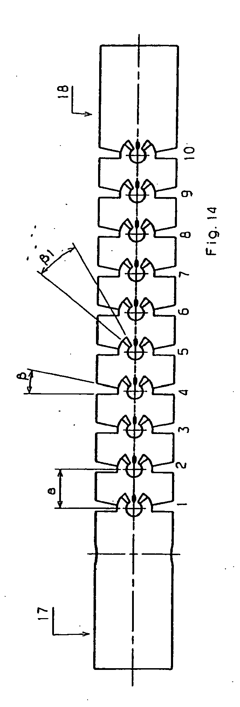

Fig. 14 zeigt einen biegbaren Abschnitt 8, der neun Rohrsegmente und zwei Endabschnitte zeigt, nämlich die neun jeweils identisch ausgebildeten Zwischen-Rohrsegmente 9 und die jeweiligen End-Rohrsegmente 17 bzw. 18, und folglich zehn Gelenkstellen aufweist. Die in dieser Figur eingezeichneten Winkel β und β¹ sowie der Abstand a von Zapfen zu Zapfen zweier benachbarter Rohrsegmente 9 können jeweils kleiner und/oder größer gewählt werden, wodurch sich die Biegbarkeit des biegbaren Abschnittes 8 ändert. Die Abmessungen dieser Winkel und Abstände können vom Fachmann je nach Wunsch festgesetzt werden.

Die vorstehend beschriebene Ausführungsform des biegbaren Abschnittes 8 ist aufgrund der jeweils um 180° versetzt angeordneten Verbindungsmittel 11 und aufgrund des Vorhandenseins von nur zwei, jeweils um 90° zu den Verbindungsmitteln über den Umfang versetzt angeordneten Steuerzügen nur in einer Ebene abbiegbar, es kann aber auch wünschenswert sein, daß der biegbare Abschnitt in einer zweiten, senkrecht auf der ersten Ebene stehenden Ebene abbiegbar sein soll, so daß letztlich eine räumliche Bewegung des biegbaren Abschnittes möglich wird.

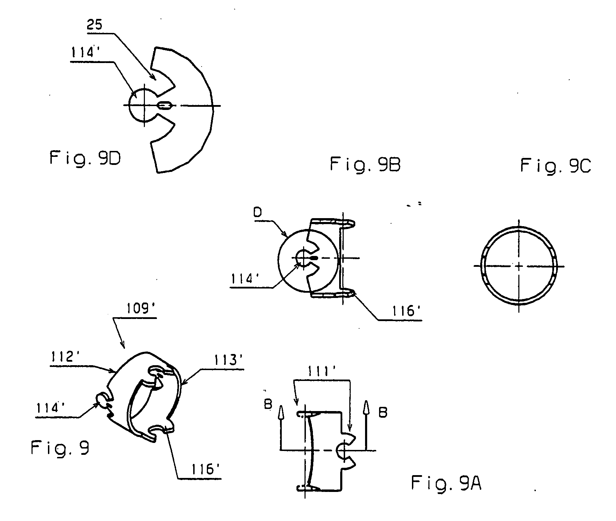

Dazu ist ein Ausführungsbeispiel anhand entsprechend ausgebildeter Rohrsegmente der äußeren und der inneren biegbaren Abschnitte in den Figuren 9 bis 10D dargestellt.

Bei diesen Rohrsegmenten 109' bzw. 109" liegen sich die an den jeweiligen Stirnseiten 112' bzw. 113' ausgebildeten Zapfen 114' bzw. Klauen 116' nicht genau gegenüber, sondern sind über den Umfang des Rohrsegmentes jeweils um 90° versetzt. Durch diese, von Rohrsegment zu Rohrsegment jeweils versetzt angeordneten Verbindungsmittel 111' kann sich nur jedes zweite Rohrsegment in einer ersten Ebene verstellen, die dazwischen liegenden anderen Rohrsegmente können sich jeweils in der zweiten Ebene verstellen. Im übrigen ist nur zu sagen, daß die Verbindungsmittel 111', also die Zapfen 114' und die Klauen 116' identisch wie bei der vorbeschriebenen ersten Ausführungsform ausgebildet sind.

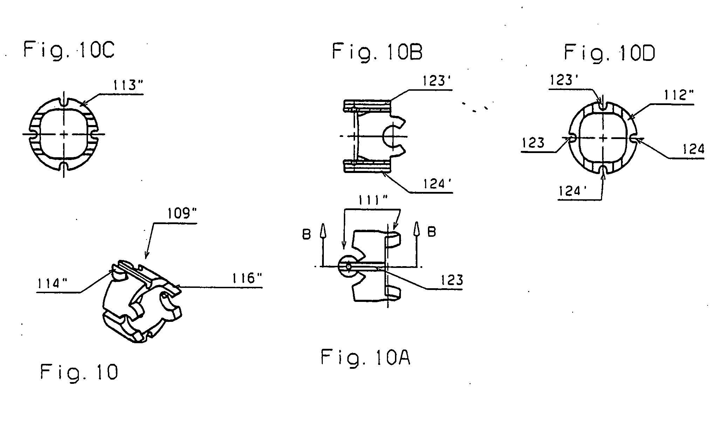

In den Figuren Fig. 10 bis Fig. 10B sind die Rohrsegmente 109" des inneren biegbaren Abschnitts 108" abgebildet, diese zeigen, daß neben den sich um 180° gegenüberliegenden axial verlaufenden Nuten 123 bzw. 124 für die Steuerzüge zur Bewegung des biegbaren Abschnittes in einer ersten Ebene jeweils um 90° versetzt zwei weitere Nuten 123' und 124' vorhanden sind, die für die Steuerzüge zur Abbiegung des biegbaren Abschnittes in der zweiten Ebene vorgesehen sind.

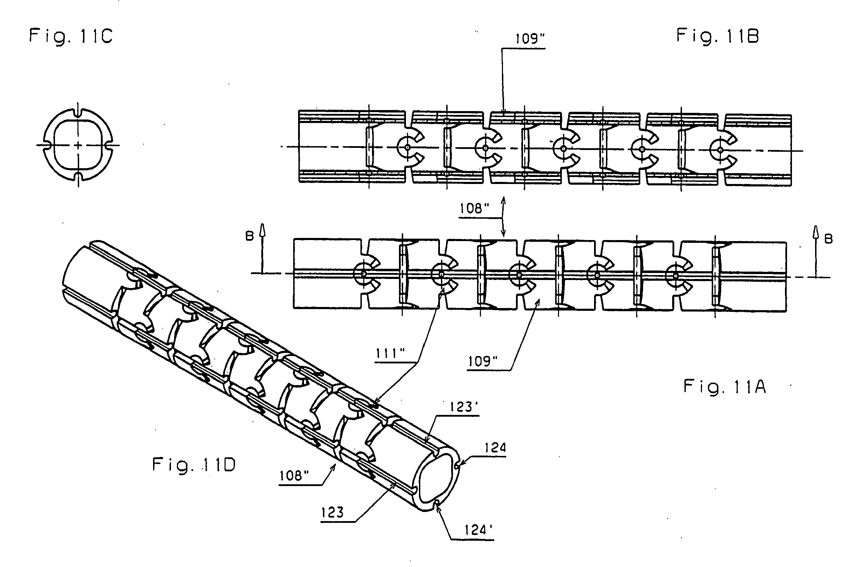

Die Figuren 11A bis 11D zeigen den aus entsprechenden Rohrsegmenten 109" zusammengesetzten inneren biegbaren Abschnitt 108". Auf eine Abbildung des dazugehörigen äußeren biegbaren Abschnittes, hergestellt aus den in den Figuren 9 bis 9C gezeigten Rohrsegmenten 109' wurde verzichtet.

Diese vorbeschriebene erfindungsgemäße Ausbildung eines biegbaren Abschnittes am distalen Ende eines Einführtubus eines Endoskopes gewährleistet gegenüber den bisher bekannten biegbaren Abschnitten solcher Endoskope eine glattwandige Ausbildung ohne vorstehende Teile und ohne Verwendung einzelner Drehzapfen, des weiteren gewährleistet die Ausbildung des biegbaren Abschnittes mit einem äußeren und einem inneren biegbaren Abschnitt eine besonders einfache Montage der Steuerzüge und gewährleistet darüber hinaus auch, daß der Innenraum des biegbaren Abschnittes glattwandig ist und daher dem Einbau erforderlicher zusätzlicher Bauteile keine Schwierigkeiten bereitet.

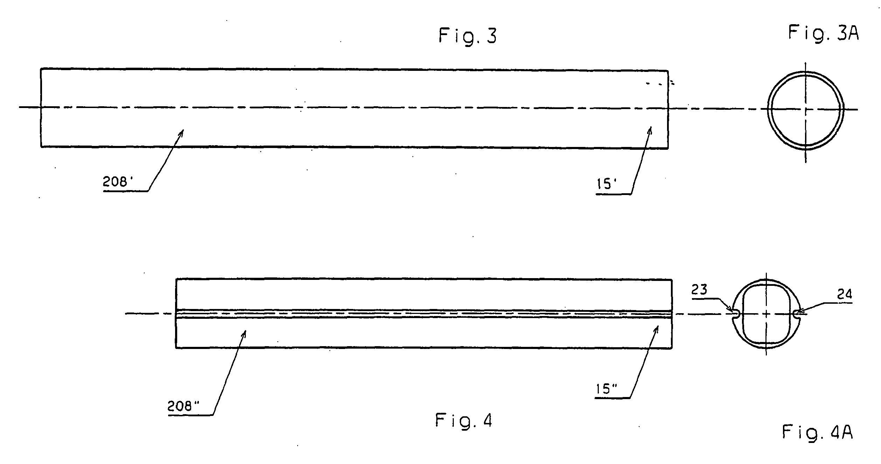

Es steht auch ein Verfahren zur Herstellung der vorbeschriebenen biegbaren Abschnitte zur Verfügung, wobei sich das Verfahren dadurch auszeichnet, daß zunächst biegesteife Rohre 208' bzw. 208", wie in den Fig. 3 und 4 dargestellt, bereitgestellt werden, die jeweils die gewünschten Außen- und Innendurchmesser aufweisen, je nach dem, ob daraus ein innerer biegbarer Abschnitt 8" öder ein äußerer biegbarer Abschnitt 8' entstehen soll. Soll ein innerer biegbarer Abschnitt 8" entstehen, weist das bereitzustellende biegesteife Rohr bereits die für die Steuerzüge erforderlichen axial verlaufenden Nuten 23, 24, 23', 24', 123, 124, 123' und 124' auf.

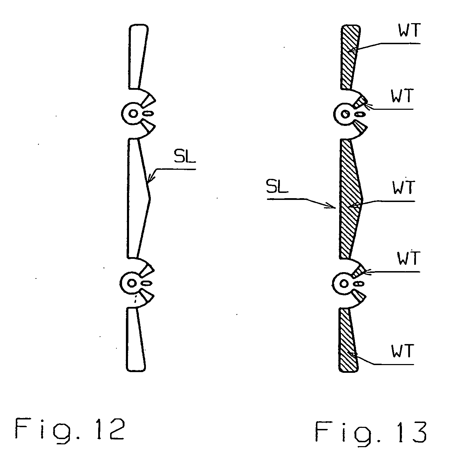

Ein solches biegesteifes Rohr 208' bzw. 208" wird in eine Laser-Schneidevorrichtung eingesetzt, wobei zwei Alternativen denkbar sind, nämlich daß das Rohr fixiert ist und die Laser-Schneidevorrichtung sich um das Rohr herumbewegt und auch gegenüber dem Rohr in axialer Richtung verschoben werden kann, oder aber die Laser-Schneidevorrichtung in einer festen Stellung angeordnet ist und das biegesteife Rohr sowohl in axialer Richtung verschoben als auch verdreht werden kann. Die Laser-Schneidevorrichtung wird dermaßen programmiert, daß entweder das Rohr gegenüber dem Laserstrahl oder aber der Laserstrahl gegenüber dem Rohr in axialer und radialer Richtung verschoben bzw. gedreht wird und der Laserstrahl auf einer Schnittlinie SL geführt wird, die die in den Figuren dargestellten und die Rohrsegmente mit den Verbindungsmitteln bildenden Abschnitte hervorbringt. In Fig. 12 ist eine solche abgewickelte Schnittlinie SL für die Laserführung dargestellt und Fig. 13 zeigt die schraffiert dargestellten und nach Durchführung des Laserschnittes auf der in Fig. 12 gezeigten Schnittlinie aus dem biegesteifen Rohr herausgeschnittenen Wandteile WT, die entweder mechanisch oder per Ultraschall herausgebrochen werden.

Aufgrund dieser Laserschnitttechnik entsteht aus dem ursprünglichen biegesteifen Rohr der gewünschte biegbare Abschnitt, wobei gewährleistet ist, daß die einzelnen Rohrsegmente des biegbaren Abschnittes sich nicht voneinander lösen können. Dies liegt daran, daß der Laserstrahl stets auf die Rohrmittelachse des biegesteifen Rohres zielt, also radial auf das Rohr gerichtet ist. Die in Fig. 8B dargestellten Schnittflächen SF speziell im Bereich der Zapfen 14 und der Klauen 16 liegen nicht parallel zueinander, sondern bilden einen Winkel zueinander. Die einzelnen Teile der Verbindungsmittel, nämlich Zapfen und Klauen könnten sich also gegeneinander nur radial nach außen verschieben, da aber auf der um 180° gegenüberliegende Seite des Rohrsegmentes ebenfalls solche Verbindungsmittel vorgesehen sind, welche sich auch nur radial nach außen verschieben könnten, werden die Rohrsegmente gegeneinander vor einem Auseinanderfallen gesichert.

## Patentansprüche

1. Biegbarer Abschnitt (8, 8', 8"), der am distalen Ende (7) eines Einführtubus (6) eines Endoskopes (1) angeordnet ist, umfassend eine Mehrzahl von Rohrsegmenten (9, 9', 9", 109', 109"), wobei jedes dieser Rohrsegmente (9, 9', 9", 109', 109") Verbindungsmittel (11, 111', 111") aufweist, die mit den Verbindungsmitteln (11, 111', 111") des benachbarten Rohrsegmentes (9, 9', 9", 109', 109") zusammenwirken, sowie Steuerzüge (10), mit denen die Abbiegung des biegbaren Abschnittes (8, 8', 8") steuerbar ist, wobei die jeweiligen Verbindungsmittel (11, 111', 111") eines Rohrsegmentes (9, 9', 9", 109', 109") an dessen Stimseiten (12, 112', 112" bzw. 13, 113', 113") in axialer Richtung vorstehend und innerhalb des Mantels (15, bzw. 15") des Rohrsegmentes (9, 9', 9", 109', 109") liegend ausgebildet sind und die Dicke des Mantels (15, 15") nicht überschreiten, wobei die Verbindungsmittel (11, 111'. 111"), die an den jeweiligen Stirnseiten (12, 112', 112" bzw. 13, 113', 113") benachbarter Rohrsegmente (9, 9', 9", 109', 109") vorgesehen sind und sich gegenüberliegen, sich nach Art einer schamierförmigen Verbindung ergänzen, **dadurch gekennzeichnet, daß** in dem biegbaren Abschnitt (8') ein hinsichtlich der Anzahl der Rohrsegmente (9') und der Anordnung der Verbindungsmittel (11) an diesen Rohrsegmenten (9') identisch ausgebildeter zweiter, innerer biegbarer Abschnitt (8') eingesetzt ist, dessen Außendurchmesser dem Innendurchmesser des äußeren biegbaren Abschnittes (8') so angepaßt ist, daß der innere biegbare Abschnitt (8") in den äußeren biegbaren Abschnitt (8') einschiebbar ist, und wobei der innere biegbare Abschnitt (8") in seiner äußeren Umfangsfläche mindestens zwei um 180° in Umfangsrichtung gegeneinander versetzt angeordnete und über die einzelnen Rohrsegmente (9", 109") in axialer Richtung verlaufende Nuten (23 bzw. 24) aufweist, in welchen jeweils ein Steuerzug (10) geführt ist, wobei die beiden Nuten (23 bzw. 24) jeweils in Umfangsrichtung um 90° versetzt gegenüber den Verbindungsmitteln (11) angeordnet sind.

2. Biegbarer Abschnitt nach Anspruch 1, **dadurch gekennzeichnet, daß** die an der einen Stirnseite (12, 112, 112) eines Rohrsegmentes (9, 9', 9", 109', 109") ausgebildeten Verbindungsmittel (11, 111', 111") nach Art eines zur Stirnseite (13, 113', 113") eines benachbarten Rohrsegmentes (9, 9', 9", 109', 109") gerichtete und vorstehende Zapfen (14. 114', 114") ausgebildet sind, und daß die an der anderen Stirnseite (13, 113', 113") des Rohrsegmentes (9, 9', 9") angeordneten Verbindungsmittel (11, 111', 111") als den Zapfen (14, 114'. 114") eines benachbarten Rohrsegmentes (9, 9', 9") umgreifende Klauen (16, 116', 116") ausgebildet sind, wobei sowohl der Zapfen (14, 114', 114") als auch die Klauen (16, 116', 116") Teile des Mantels (15', 15") des jeweiligen Rohrsegmentes (9, 9', 9", 109', 109") sind.

3. Biegbarer Abschnitt nach Anspruch 2, **dadurch gekennzeichnet, daß** die jeweils an einer der Stirnseiten (12, 112', 112" bzw. 13, 113', 113") eines Rohrsegmentes (9, 9', 9", 109', 109") angeordneten Verbindungsmittel (11,111', 111") entweder als Zapfen (14, 114', 114") oder als Klauen (16, 116', 116") ausgebildet sind, wobei dann die an der anderen Stirnseite (13, 113', 113" bzw. 14, 114', 114") des selben Rohrsegmentes (9, 9', 9", 109', 109") vorgesehenen Verbindungsmittel (11, 111', 111") entweder als Klauen (16, 116'. 116") oder als Zapfen (14, 114', 114") ausgebildet sind.

4. Biegbarer Abschnitt nach Anspruch 2, **dadurch gekennzeichnet, daß** der an der Stirnseite (12, 112', 112") eines Rohrsegmentes (9, 9', 9", 109', 109") angeordnete Zapfen (14, 114', 114") nach Art- einer kreisförmigen Scheibe ausgebildet ist und daß die an der Stirnseite (13, 113', 113") des benachbarten Rohrsegmentes (9, 9', 9", 109', 109") vorgesehenen Klauen (16, 116', 116") eine kreisförmige Ausnehmung zwischen sich einschließen, welche die Abmessungen des kreisscheibenförmigen Zapfens (14, 114', 114") aufweist, und wobei die Klauen (16, 116', 116") den Zapfen (14, 114', 114") in dieser Ausnehmung aufnehmen.

5. Biegbarer Abschnitt nach einem der vorstehenden Ansprüche, **dadurch gekennzeichnet, daß** das am proximalen Ende des biegbaren Abschnittes (8, 8', 8") angeordnete erste Rohrsegment (17'. 17") und das am distalen Ende des biegbaren Abschnittes (8, 8', 8") angeordnete letzte Rohrsegment (18', 18") jeweils an ihren die Enden (19', 19" bzw. 20', 20") des biegbaren Rohrsabschnitts bildenden Stimseiten (21'. 21" bzw. 22',22") keine Verbindungsmittel nach Art von Zapfen und/oder Klauen aufweisen.

6. Biegbarer Abschnitt nach einem der obigen Ansprüche, **dadurch gekennzeichnet, daß** an der äußeren Umfangsfläche des inneren biegbaren Abschnitts (8") vier axial verlaufende Nuten (123, 123'; 124, 124') vorgesehen sind, die jeweils um 90° in Umfangsrichtung zueinander versetzt angeordnet sind, wobei gleichzeitig die an den jeweiligen Stimseiten (112', 112" bzw. 113', 113") eines Rohrsegmentes (109', 109") paarweise angeordneten Verbindungsteile (111', 111") um 90° zueinander versetzt sind.

7. Biegbarer Abschnitt nach einem der obigen Ansprüche, **dadurch gekennzeichnet, daß** die einzelnen Rohrsegmente (9, 9', 9", 109', 109") der inneren und der äußeren biegbaren Abschnitte (8, 8' bzw. 8") im Zustand ihrer vollständigen Kongruenz zueinander, jeweils miteinander durch eine Punktschweißung aneinander fixiert sind.

## Claims

1. A bendable portion (8, 8', 8"), which is arranged at the distal end (7) of an insertion tube (6) of an endoscope (1), said portion comprising a plurality of tube segments (9, 9', 9", 109', 109"), each of said tube segments (9, 9', 9", 109', 109") having connecting means (11, 111', 111 ") which cooperate with the connecting means (11, 111', 111 ") of the adjacent tube segment (9, 9', 9", 109', 109"), and further comprising control wires (10), using which the bending of the bendable portion (8, 8', 8") can be controlled, the respective connecting means (11, 111', 111") of a tube segment (9, 9', 9", 109', 109") being provided such that they axially protrude from its faces (12, 112', 112" and 13, 113', 113", respectively) and are located within the mantle (15, or 15", respectively) of the tube segment (9, 9', 9", 109', 109") and such that they do not exceed the thickness of the mantle (15, 15"), the connecting means (11, 111', 111") provided on the respective faces (12, 112', 112" and 13, 113', 113", respectively) of adjacent tube segments (9, 9', 9", 109', 109") and located opposite each other complementing each other in the manner of a hinge-type connection, **characterised in that** the bendable portion (8') has inserted therein a second, inner bendable portion (8"), which is of identical design as regards the number of tube segments (9') and the arrangement of the connecting means (11) on said tube segments (9'), the outer diameter of said inner bendable portion (8") being adapted to the inner diameter of the outer bendable portion (8') such that the inner bendable portion (8") is slidable into the outer bendable portion (8'), and said inner bendable portion (8") comprising at least two grooves (23 and/or 24) in its outer peripheral surface, which have a circumferential offset of 180° between them and extend in an axial direction over the individual tube segments (9", 109"), said grooves each having a control wire (10) guided therein and both of said grooves (23 and 24, respectively) being arranged with a circumferential offset of 90° relative to the connecting means (11).

2. The bendable portion according to claim 1, **characterised in that** the connecting means (11, 111', 111") provided on one face (12, 112', 112") of a tube segment (9, 9', 9", 109', 109") are designed in the manner of a pin (14, 114', 114") directed and protruding towards the face (13, 113', 113") of an adjacent tube segment (9, 9', 9", 109', 109"), and that the connecting means (11, 111', 111") arranged on the other face (13, 113', 113") of the tube segment (9, 9', 9") are provided as claws (16, 116', 116") embracing the pin (14, 114', 114") of an adjacent tube segment (9, 9', 9"), both the pin (14, 114', 114") and the claws (16, 116', 116") being parts of the mantle (15', 15") of the respective tube segment (9, 9', 9", 109', 109").

3. The bendable portion according to claim 2, **characterised in that** the connecting means (11, 111', 111") provided on one of the faces (12, 112', 112" and 13, 113', 113", respectively) of a tube segment (9, 9', 9", 109', 109") are provided either as a pin (14, 114', 114") or as claws (16, 116', 116"), in which case the connecting means (11, 111', 111 ") provided on the other face (13, 113', 113" and 14, 114', 114", respectively) of the same tube segment (9, 9', 9", 109', 109") are then either provided as claws (16, 116', 116") or as a pin (14, 114', 114").

4. The bendable portion according to claim 2, **characterised in that** the pin (14, 114', 114") arranged on the face (12, 112', 112") of a tube segment (9, 9', 9", 109', 109") is designed in the manner of a circular disk and that the claws (16, 116', 116") provided on the face (13, 113', 113") of the adjacent tube segment (9, 9', 9", 109', 109") enclose a circular recess between them, said recess having the dimensions of the circular disk-shaped pin (14, 114', 114"), and wherein the claws (16, 116', 116") accommodate the pin (14, 14', 14") in said recess.

5. The bendable portion according to any one of the preceding claims, **characterised in that** the first tube segment (17', 17") at the proximal end of the bendable portion (8, 8', 8") and the last tube segment (18', 18") at the distal end of the bendable portion (8, 8', 8") respectively have no pin- and/or claw-type connecting means on their faces (21', 21" and 22', 22", respectively) forming the ends (19', 19" and 20', 20", respectively) of the bendable tube portion.

6. The bendable portion according to any one of the above claims, **characterised in that** four axially extending grooves (123, 123'; 124, 124') are provided on the outer peripheral surface of the inner bendable portion (8"), which grooves respectively have a circumferential offset of 90° between them, and at the same time, the connecting parts (111', 111") arranged in pairs on the respective faces (112', 112" and 113', 113", respectively) of a tube segment (109', 109") have an offset of 90° between them.

7. The bendable portion according to any one of the above claims, **characterised in that** the individual tube segments (9, 9', 9", 109', 109") of the inner and outer bendable portions (8, 8' and 8", respectively) are fixed to each other by spot welding in a state of complete congruence with each other.

## Revendications

1. Partie flexible (8, 8', 8"), disposée à l'extrémité distale (7) d'un tube d'introduction (6) d'un endoscope (1), comportant une pluralité de segments tubulaires (9, 9', 9", 109', 109"), chacun de ces segments tubulaires (9, 9', 9", 109', 109") comportant des moyens d'assemblage (11, 111', 111") qui coopèrent avec les moyens d'assemblage (11, 111', 111") d'un segment tubulaire (9, 9', 9", 109', 109") adjacent, ainsi que des câbles de commande (10), qui permettent de commander la courbure de la partie flexible (8, 8', 8"), les moyens d'assemblage (11, 111', 111") d'un segment tubulaire (9, 9', 9", 109', 109") étant réalisés en forme de saillie axiale sur les faces frontales (12, 112', 112" et 13, 113', 113") de celui-ci et disposés à l'intérieur de l'enveloppe (15 et 15") du segment tubulaire (9, 9', 9", 109', 109") et ne dépassant pas l'épaisseur de l'enveloppe (15, 15"), les moyens d'assemblage (11, 111', 111"), qui sont prévus et se font face sur les faces frontales (12, 112', 112" et 13, 113', 113") respectives de segments tubulaires (9, 9', 9", 109', 109") adjacents, se complétant à la manière d'un assemblage à charnière, **caractérisée en ce que** dans la partie flexible (8') est insérée une deuxième partie flexible (8") intérieure, qui est conçue de manière identique en ce qui concerne le nombre de segments tubulaires (9') et l'agencement des moyens d'assemblage (11) sur ces segments tubulaires (9') et dont le diamètre extérieur est adapté au diamètre intérieur de la partie flexible (8') extérieure, de telle sorte que la partie flexible (8") intérieure peut être introduite dans la partie flexible (8') extérieure, et la partie flexible (8") intérieure comportant dans sa face périphérique au moins deux rainures (23 et 24), qui sont décalées de 180° l'une par rapport à l'autre et s'étendent dans le sens axial sur les différents segments tubulaires (9", 109") et dans chacune desquelles est guidé un câble de commande (10), les deux rainures (23 et 24) étant disposées chacune dans le sens périphérique en étant décalées de 90° par rapport aux moyens d'assemblage (11).

2. Partie flexible selon la revendication 1, **caractérisée en ce que** les moyens d'assemblage (11, 111', 111"), disposés sur l'une des faces frontales (12, 112', 112") d'un segment tubulaire (9, 9', 9", 109', 109") sont réalisés sous la forme d'un téton (14, 114', 114") en saillie et orienté vers la face frontale (13, 113', 113") d'un segment tubulaire (9, 9', 9", 109, 109') adjacent, et **en ce que** les moyens d'assemblage (11, 111', 111"), disposés sur l'autre face frontale (13, 113', 113") du segment tubulaire (9, 9', 9") sont réalisés sous forme de pinces (16, 116', 116") enserrant le téton (14, 114', 114") d'un segment tubulaire (9, 9', 9") adjacent, le téton (14, 114', 114") et les pinces (16, 116', 116") étant des parties de l'enveloppe (15', 15") du segment tubulaire (9, 9', 9", 109', 109") respectif.

3. Partie flexible selon la revendication 2, **caractérisée en ce que** les moyens d'assemblage (11, 111', 111"), disposés sur l'une des faces frontales (12, 112', 112" ou 13, 113', 113") d'un segment tubulaire (9, 9', 9", 109', 109"), sont réalisés soit sous forme de téton (14, 114', 114"), soit sous forme de pinces (16, 116', 116"), les moyens d'assemblage (11, 111', 111"), disposés dans ce cas sur l'une face frontale (13, 113', 113" ou 12, 112', 112") du même segment tubulaire (9, 9', 9", 109', 109"), étant réalisés soit sous forme de pinces (16, 116', 116"), soit sous forme de téton (14, 114', 114").

4. Partie flexible selon la revendication 2, **caractérisée en ce que** le téton (14, 114', 114"), disposé sur la face frontale (12, 112', 112") d'un segment tubulaire (9, 9', 9", 109', 109") est conçu sous forme de disque circulaire et **en ce que** les pinces (16, 116', 116") prévues sur la face frontale (13, 113', 113") du segment tubulaire (9, 9', 9", 109', 109") adjacent forment entre elles un évidement circulaire, qui a les dimensions du téton (14, 114', 114") en forme de disque circulaire, et les pinces (16, 116', 116") recevant le téton (14, 114', 114") dans cet évidement.

5. Partie flexible selon l'une quelconque des revendications précédentes, **caractérisée en ce que** le premier segment tubulaire (17', 17"), disposé sur l'extrémité proximale de la partie flexible (8, 8', 8") et le dernier segment tubulaire (18', 18"), disposé sur l'extrémité distale de la partie flexible (8, 8', 8"), ne comportent pas de moyen d'assemblage sous forme de téton et/ou de pinces au niveau de leurs faces frontales (21', 21" et 22', 22") formant les extrémités (19', 19" et 20', 20") de la partie tubulaire flexible.

6. Partie flexible selon l'une quelconque des revendications précédentes, **caractérisée en ce que** sur la face périphérique extérieure de la partie flexible (8") intérieure sont prévues quatre rainures (123, 123' ; 124, 124') orientées dans le sens axial, qui sont disposées en étant décalées de 90° les unes par rapport aux autres dans le sens périphérique, les moyens d'assemblage (111', 111"), agencés par paires sur les faces frontales (112', 112" et 113', 113") respectives d'un segment tubulaire (109', 109") étant également décalés de 90° les uns par rapport aux autres.

7. Partie flexible selon l'une quelconque des revendications précédentes, **caractérisée en ce que** les différents segments tubulaires (9, 9', 9", 109', 109") de la partie flexible (8, 8' et 8") intérieure et extérieure, à l'état de leur totale congruence, sont fixés respectivement les uns contre les autres au moyen d'un point de soudage.
